# EUROPÄISCHE PATENTSCHRIFT

(11) **EP 0 522 999 B1**
(45) Veröffentlichungstag und Bekanntmachung des Hinweises auf die Patenterteilung: **31.07.1996**
(21) Anmeldenummer: 92810427.2
(22) Anmeldetag: 04.06.1992
(51) Int. Cl.: A61F 2/38, A61F 2/30

(54) **Patella-Prothese**
Patella prosthesis
Prothèse de rotule

(30) Priorität: 05.07.1991 CH 2004/91
(43) Veröffentlichungstag der Anmeldung: 13.01.1993
(73) Patentinhaber: Sulzer Orthopädie AG, 6340 Baar (CH)
(72) Erfinder: Gschwend, Norbert, Prof. Dr. med., CH-8008 Zürich (CH); Koch, Rudolf, CH-8500 Frauenfeld (CH)
(74) Vertreter: Heubeck, Bernhard

(56) Entgegenhaltungen:
- EP-A- 0 338 976
- EP-A- 0 420 795
- FR-A- 2 653 992
- US-A- 4 997 445

## Beschreibung

Die Erfindung zeigt eine Patella-Prothese, die aus einem künstlichen Gleitkörper mit Gleitfläche besteht, der an der Rückseite der Patella mittels einer Verankerung anbringbar ist, wobei die Gleitfläche aus einem Polyethylenkörper gebildet ist, welcher eine Metalldrahtschicht eines Verankerungselementes in sich aufnimmt, wobei das Verankerungselement aus einer metallenen Sandwichkonstruktion mit einem mittleren Trägerteil und beidseitig damit verbundenen Metalldrahtschichten besteht, und wobei Verankerungsmittel zur Primärverankerung vom Trägerteil durch Aussparungen der patellaseitigen Metalldrahtschicht herausragen.

Die Patentschrift US 4,479,271 zeigt eine Patella-Prothese, bei der die primäre Verankerung durch Zapfen vorgenommen wird, welche aus einem Metallkern bestehen, der von einer porösen Faserhülse umgeben ist, die ihrerseits aussen und innen massgenau auf einem nichtmetallischen Zapfen aus Polyethylen steckt. Die Aussparungen für die Zapfen müssen in der Patella eingearbeitet werden und sollten mit ihren Achsen senkrecht zur Aufnahmeebene für die eigentliche Aufnahmefläche liegen, um deren Anliegen und späteres Einwachsen zu ermöglichen. Achsabstand und Lage der Aufnahmebohrungen in der Patella müssen ebenfalls sehr genau sein.

Die Patentschrift US 4 997 445 offenbart eine Patella-Prothese, die mit ihrer Rückseite auf der zurückgeschnittenen Innenseite einer Patella befestigbar ist und die einen mit Zapfen und Vorsprüngen versehenen Trägerkörper aufweist. Der Trägerkörper ist auf der Rückseite in seiner Kontur von Stirnfläche und Zapfen zurückgesetzt, um in einem Sintervorgang Schichten aus einem wirren gesinterten Drahtfilz zu befestigen. Auf der Vorderseite des Trägerkörpers sind schwalbenschwanzförmige Vorsprünge angebracht und ist ein Gitter aufgesintert, damit ein Gleitkörper aus Polyethylen darum geformt werden kann. Dieser Prothesenaufbau ist aufwendig in der Herstellung. Schon der Trägerkörper ist relativ kompliziert in seiner Form und die Verankerung vom Polyethylen am Trägerkörper setzt voraus, dass die gesamte Kunststoffmasse beim Verankern fliessfähig ist und dass entsprechende Formen vorhanden sind. Es ist daher nicht möglich, einen aus einem geprüften Rohling fertig geschnittenen Gleitkörper zu verwenden und in einer Fügeoperation anzusetzen. Im weiteren müssen Formen für die Herstellung der wirr gesinterten Schicht aus Drahtfilz hergestellt werden, deren Abmessungen den Massen an dem Trägerkörper entsprechen. Der Sintervorgang selbst setzt eine gleichmässige Erwärmung und das Einhalten bestimmter Temperaturen voraus, was ein Indiz für lange Fertigungszeiten ist.

Hier schafft die Erfindung Abhilfe. Sie löst die Aufgabe eine kostengünstige und einfach verankerbare Patella-Prothese herzustellen. Diese Aufgabe wird mit den Kennzeichen vom unabhängigen Anspruch gelöst.

Die Vorteile der Erfindung sind darin zu sehen, dass auf der Patella als Vorbereitung nur eine ebene Auflagefläche geschaffen werden muss, dass die Prothese eine hohe Eigensteifigkeit und Formbeständigkeit aufweist und dass die Begrenzungsfläche zum einwachsenden Knochengewebe aus Metall besteht. Im weiteren ergeben sich fertigungstechnische Vorteile aus dem Aufbau der Prothese, indem ein Verankerungsteil in reiner Blechbearbeitungstechnik geschaffen wird und in einer einfachen Fügeoperation der Gleitkörper daran befestigt wird.

Zur Primärbefestigung der Prothese genügt es ein in sich ebenes Flächenstück auf der Rückseite der Patella herzustellen. Dies kann beispielsweise durch eine Flachraspel erfolgen, wobei die genaue Winkellage des ebenen Flächenstücks sekundäre Bedeutung hat, da die Gleitfläche der Prothese aus einem Kugelausschnitt gebildet ist. Die Prothese wird vom Operateur am richtigen Ort aufgesetzt und in die Patella zur Primärverankerung eingepresst. Messerartige Blechschenkel bilden eine selbsthemmende Primärverankerung im Knochengewebe, ohne dieses zu schädigen. Der Verlust an Knochengewebe ist gering.

Im folgenden wird die Erfindung anhand von einem Ausführungsbeispiel beschrieben. Es zeigt:
- Fig. 1: den Längsschnitt durch eine Patella mit einer erfindungsgemässen Prothese, und
- Fig. 2: die Ansicht auf die der Patella zugekehrte Unterseite der Prothese gemäss Figur 1.

In den Figuren ist eine Patella-Prothese gezeigt, die aus einem künstlichen Gleitkörper 2 aus Polyethylen mit Gleitfläche 3 besteht, wobei der Gleitkörper 2 an der Rückseite der Patella 1 mittels einer Verankerung angebracht ist. Die Gleitfläche 3 besteht aus dem Ausschnitt einer Polyethylenkugel, welcher ein Metallgitternetz 4 eines Verankerungselementes 9 in sich aufnimmt. Das Verankerungselement besteht aus einer metallenen Sandwichkonstruktion mit einem mittleren Trägerblech und beidseitig aufgepunkteten Metallgitternetzen 4, 6, wobei zugeschärfte Blechschenkel 7 zur Primärverankerung vom Trägerblech 5 durch Aussparungen 8 des patellaseitigen Metallgitternetzes 6 herausragen.

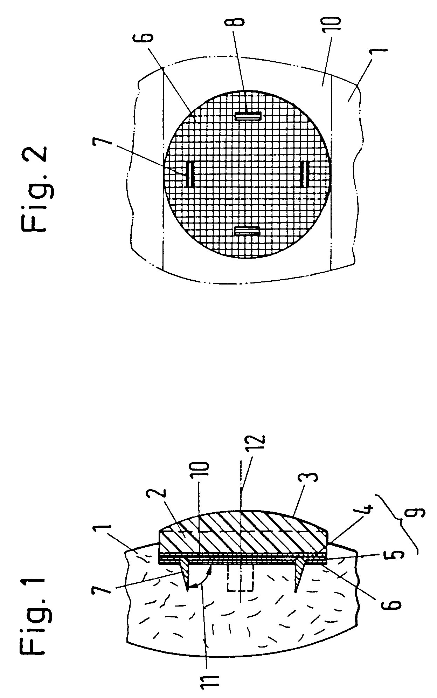

Aus Figur 1 ist die Sandwichkonstruktion des Verankerungselementes 9 ersichtlich, das aus einem mittleren Titanblech 5 mit einer Wandstärke von 0,5 mm und beidseitig angepunkteten Gitternetzen 4, 6 aus Titan besteht. Das Verankerungselement 9 ist als Rondelle mit einem Durchmesser von 27 mm zugeschnitten. Der Gleitkörper 2 besteht aus einer Rondelle mit gleichem Durchmesser und schliesst mit einer kugelförmigen Gleitfläche 3 ab, die einen Radius von 30 mm und einen Mittelpunkt in der Achse 12 der Rondellen aufweist. Das Metallgitternetz 4 zum Gleitkörper 2 durchdringt diesen und ist bis an das Trägerblech 5 von Polyethylen umschlossen.

In Figur 2 weist das patellaseitige Metallgitternetz 6 vier rechteckige Aussparungen 8 in der Orientierung des Gitters auf, durch die jeweils ein zugeschärfter Blechschenkel von 4 mm Breite um 6 mm vom Trägerblech 5 vorsteht. Die Blechschenkel 7 sind mit einem Stanzwerkzeug (nicht gezeigt) entlang der vorstehenden Kontur aus dem Trägerblech 5 freigeschnitten worden und um einen Winkel 11 von 90° aus der Ebene des Trägerblechs 5 herausgebogen worden.

Die Verankerung erfolgt an der Innenseite der Patella auf einer in sich ebenen Verankerungsfläche 10, die mit einer Flachraspel (nicht gezeigt) quer zum Sehnenverlauf erzeugt wurde. Die Prothese wird in einer um die Länge der vorstehenden Blechschenkel 7 korrigierten Lage an der Patella 1 aufgesetzt und in die Endlage eingepresst. Die messerartigen Blechschenkel 7 graben sich in das Knochengewebe ein und bilden unter Selbsthemmung im verdrängten Knochengewebe eine Primärverankerung für die ganze Prothese. Das Knochengewebe kann ungehindert bis zum Trägerblech 5 durch das patellaseitige Metallgitternetz 6 einwachsen. Das Trägerblech 5 ist in sich so steif, dass Kräfte an den Berührungspunkten der Gleitfläche 3 gleichmässig auf das im Metallgitternetz 6 eingewachsene Knochengewebe übertragen werden.

## Patentansprüche

1. Patella-Prothese bestehend aus einem künstlichen Gleitkörper (2) mit Gleitfläche (3), der an der Rückseite der Patella (1) mittels einer Verankerung anbringbar ist, wobei die Gleitfläche (3) aus einem Polyethylenkörper gebildet ist, welcher eine Metalldrahtschicht (4) eines Verankerungselementes (9) in sich aufnimmt, wobei das Verankerungselement (9) aus einer metallenen Sandwichkonstruktion mit einem mittleren Trägerteil (5) und beidseitig damit verbundenen Metalldrahtschichten (4, 6) besteht, und wobei Verankerungsmittel (7) zur Primärverankerung vom Trägerteil (5) durch Aussparungen (8) der patellaseitigen Metalldrahtschicht (6) herausragen, dadurch gekennzeichnet, dass die Gleitfläche (3) aus dem Ausschnitt einer Polyethylenkugel besteht und der Trägerteil (5) ein Trägerblech ist, auf welches beidseitig die als Metallgitternetze ausgebildeten Metalldrahtschichten (4, 6) aufgepunktet sind, und dass die Verankerungsmittel (7) zugeschärfte Blechschenkel sind, welche als Bestandteil des Trägerblechs durch ein Stanzwerkzeug freigeschnitten und aus der Ebene des Trägerblechs (5) um einen Winkel (11) von 90° herausgebogen sind.

## Claims

1. A patella prosthesis consisting of an artificial slide member (2) having a slide surface (3), which member can be mounted at the rear of the patella (1) by means of an attachment, wherein the slide surface (3) is formed of a polyethylene member which contains a metal wire layer (4) of an attachment element (9), wherein the attachment element (9) consists of a metal sandwich construction having a central support part (5) and metal wire layers (4, 6) connected therewith on both sides, and wherein attachment means (7) for the primary attachment of the support part (5) protrude through recesses (8) in the metal wire layer (6) on the side of the patella,
**characterised in that** the slide surface (3) consists of the sector of a polyethylene sphere and the support part (5) is a support plate on which metal wire layers (4, 6) constructed as metal grids are spot welded on both sides,
**and in that** the attachment means (7) are bevelled sheet metal arms, which as a component of the support sheet are cut free by a blanking die and bent out of the plane of the support sheet (5) by an angle of 90°.

## Revendications

1. Prothèse de rotule constituée d'un corps glissant (2) artificiel avec surface de glissement (3), qui peut être appliquée au moyen d'un ancrage sur la face arrière de la rotule (1), la surface de glissement (3) étant formée dans un corps en polyéthylène, qui loge en soi une couche de fil métallique (4) d'un élément d'ancrage (9), l'élément d'ancrage (9) étant constitué d'une construction sandwich en métal avec un élément de support (5) central et des couches de fil métallique (4, 6) reliées à celui-ci des deux côtés, et des moyens d'ancrage (7) pour l'ancrage primaire de l'élément de support (5) ressortant à travers des découpes (8) de la couche de fil métallique (6) côté rotule, caractérisée en ce que la surface de glissement (3) est constituée de la découpe de sphère de polyéthylène et l'élément de support (5) est une tôle de support sur laquelle sont pointées des deux côtés les couches de fil métallique (4, 6) configurées en grille métallique, et en ce que les moyens d'ancrage (7) sont des branches de tôle affilées, qui en tant que partie intégrante de la tôle de support sont dégagées par un outil de découpe et repliées hors du plan de la tôle de support (5), d'un angle (11) de 90°.
